# EUROPEAN PATENT SPECIFICATION

(11) **EP 0 539 474 B1**
(45) Date of publication and mention of the grant of the patent: **11.10.1995**
(21) Application number: 91913520.2
(22) Date of filing: 16.07.1991
(51) Int. Cl.: C11D 9/00, C11D 9/22

(54) **RINSE-FREE SHAMPOO**
TROCKENSHAMPOO
SHAMPOOING SEC

(30) Priority: 17.07.1990 US 553864
(43) Date of publication of application: 05.05.1993
(73) Proprietor: MICRO VESICULAR SYSTEMS, INC., Nashua New Hampshire 03063 (US)
(72) Inventor: WEINSTEIN, Benjamin, Vineland, NJ 08360 (US); WALLACH, Donald, F., H., Hollis, NH 03049 (US)
(74) Representative: Holdcroft, James Gerald, Dr.
(86) International application number: US9105025
(87) International publication number: WO9201777

(56) References cited:
- EP-A- 0 365 845
- DE-A- 1 911 143
- US-A- 3 969 280
- US-A- 4 160 063
- US-A- 4 394 520
- US-A- 4 802 997
- PATENT ABSTRACTS OF JAPAN vol. 11, no. 369 (C-461)2 December 1987

## Description

The present invention relates to a new cleansing agent such as a shampoo. More particularly, this invention concerns a rinse-free shampoo which leaves less residue than conventional rinse-free shampoos and has a higher aqueous content.

Convention shampoos are made, primarily, of water, cleansing agents such as sodium dodecyl sulfate, thickeners, foam boosters, and fragrance. While these shampoos provide perfectly acceptable hair cleansing in most circumstances, they require a great deal of water to rinse the shampoo out of the hair. In most circumstances this is acceptable but rinsing is a serious problem for the infirm, e.g., geriatric patients and those otherwise hospitalized. While appearance is still an important factor to these people, they are often bedridden so they cannot easily use conventional means of washing their hair. Accordingly, "rinse-free" shampoos were developed several years ago. Rinse-free shampoos are used by rubbing into the hair, thereby entrapping or chelating the dirt particles and oils, and are removed by toweling and/or combing or brushing the hair. The term "rinse-free shampoo," as used herein, means a shampoo which requires little or no additional liquid for its operation.

Although the early rinse-free shampoos had some success, they has two major problems: first, they did not clean hair as well as conventional shampoos; and second, they were apt to leave a large amount of residue on the hair. This residue, which may include chelated dirt, particulates from the shampoo itself, or other particulate forms, leaves the hair with a dull, unclean appearance, prevents the pleasing appearance sought by the consumer, and can lead to itchiness and scratching of the scalp. Further, the early shampoos washed away many of the natural oils.

The early rinse-free shampoos had sodium lauryl sulfate as their primary ingredient since the addition of ammonium ions gave problems with residue. Present rinse-free shampoos use materials such as triethanolamide lauryl sulfate, propylene glycol, and a small amount of cocamide diethanolamine. However, these formula modifications have not solved the problems caused by the residue, particularly the resultant greasy and/or gritty feeling of the hair.

Accordingly, an object of the invention is to provide a rinse-free shampoo which is inexpensive yet solves the problems of residue build-up and provides a clean, shiny appearance to the hair.

A further object of the invention is to provide a biodegradable rinse-free shampoo which can be used for camping or other purposes where a conventional shampoo is not convenient.

A further object of the invention is to provide a shampoo for geriatric and other infirm patients.

These and other objects and features of the invention will be apparent from the following description.

### Summary of the Invention

The present invention features a personal cleaner such as a shampoo which contains a gel-like absorbent and has high water content. When used as a shampoo, it can act in a rinse-free mode without leaving significant residue on the hair, thus provides better cleansing action than conventional rinse-free shampoos.

The personal cleaner of the invention has its primary ingredients a carboxylated cellulosic material which is cross-linked and stabilized by an organo-metallic cross-linking agent, a soap, and water. The personal cleaner is in the form of a shampoo, preferably a rinse-free shampoo. The carboxylated cellulosic material is carboxymethylcellulose, preferably a carboxymethylcellulose having a high DS (Degree of Substitution) value, most preferably 0.5 or greater. Preferred organo-metallic cross-linking agents are those which contain an aluminum complex such as aluminum acetate stabilized by boric acid. While other metallic ions such as ferric ions may be used, the aluminum ions appear to provide the best cross-linking.

The soap is a lauric acid derivative selected from lauric acid itself, sodium lauryl sulfate, ammonium lauryl sulfosuccinate, lauramides, lauramide diethanolamine, lauryl betaines, and mixtures thereof.

The shampoo has a high aqueous content, with water providing more than 90% by weight of the shampoo. This high water content and the resultant low solid content allows proper cleaning while leaving little residue on the hair.

Further features on the invention will be explained in connection with the following description.

### Detailed Description of the Invention

The shampoo of the present invention provides excellent cleaning action without residue build-up even in circumstances where it is used in a rinse-free mode, e.g., there is no water rinse used to remove the shampoo. This is accomplished by the high water content (low solid content) of the shampoo and the absorbent gel which entraps the dirt without leaving residue. This high water content means that the shampoo and the entrapped dirt are easily removed by toweling and/or combing the hair.

The shampoo of the invention is based on the use of a cross-linked, carboxylated cellulosic material which acts as an absorbent. This absorbent has a high capacity for holding aqueous solutions so that a higher water content can be used without making the shampoo "runny." This absorbent is biodegradable and substantially nonallergenic. Further details concerning this absorbent are described in United States Patent Application Serial No. 320,944 (US 4959 341) and United States Patent Application Serial No. 410,356 (US 4952 550).

The shampoo may contain a variety of other materials including stabilizers, conditioners, foam boosters, and fragrances.

The following non-limiting example will further illustrate the invention.

### Example

In this Example, a "rinse-free" shampoo was made using primarily carboxymethylcellulose, sodium ammonium lauryl sulfosuccinate, and sodium lauric betaine, and water. This shampoo is stable and only a small amount, e.g., a teaspoon is needed to be added to the hair for cleansing purposes.

The shampoo was made as a two-part formulation to obtain the best product. While this procedure is not entirely necessary, it ensures limited air entrapment and stabilization of heat labile materials.

First, the carboxymethylcellulose ("CMC"), preferably a high DS CMC such as Aqualon 7HF, is dry blended with a cross-linking agent, e.g., aluminum acetate stabilized with boric acid, by geometric dilution to insure complete mixing. In the example of the formulation, 1.5% Aqualon 7HF was blended with 0.035% aluminum acetate stabilized with boric acid in a dry vessel. (All percentages are approximate and based on the final weight of the shampoo.) Deionized water (96.5%) is added to a jacketed stainless steel kettle equipped with a blade mixer and is heated to 70-80° C. while stirring. e.g., by passing steam through the jacket. Two preservatives, methyl paraben (0.2%) and propyl paraben (0.03%), are added to the water. Conditioners such as d-panthanol, ceytl alcohol, and stearyl alcohol may also be added at 0.3-0.5% of the total weight. The solution is stirred until the preservatives are completely dissolved. The resulting solution is then transferred to another stainless steel vessel fitted with an homogeneous mixer and is allowed to cool until approximately 60° C. The CMC/cross-linker powder is then added at high speed mixing to insure there is no lump formation and mixed until homogeneous. This resulting material is allowed to sit, e.g., overnight, to minimize the air bubbles in the final product. The CMC is cross-linked by the aluminum acetate/borate and swells with the aqueous solution.

The other part of the shampoo uses a mixture of cleansers, foam boosters, thickeners, conditioners, and fragrance to yield the cleaning action and a more "shampoo-like" texture. Approximately 0.7% of a 40% solution of ammonium lauryl sulfosuccinate in water (Monamate LNT 40-MONA), approximately 0.125% lauramide diethanolamine (Monamid 1089-MONA), 0.375% sodium lauryl betaine (Monateric 985A-MONA), 0.5% propylene glycol, and 0.075% fragrance are blended together. The Monamate LNT 40 and Monateric 985A are soaps, e.g., amphoteric surfactants, while the Monamid 1089 is a foam booster which also may act as a conditioner. The propylene glycol is used primarily as a thickener so as to give a more conventional shampoo-like consistency. Once these ingredients are all blended, they are added to the cross-linked CMC-water mixture at slow speed and mixed slowly to ensure that there is no air entrapment.

For use the shampoo is a rinse-free mode, approximately a teaspoon of the shampoo is put in the hair and rubbed in vigorously. After being allowed to set for several minutes, the shampoo is removed by toweling and/or brushing or combing, leaving clean, manageable hair. The shampoo also could be used as a conventional shampoo.

Other materials and embodiments useful in the present invention are known by those skilled in the art. For example, although the primary use of the cleaner is as a shampoo, it can be used to remove dirt or oil from hands or other surfaces. Accordingly, such other materials and embodiments thereof are included within the following claims.

## Claims

1. A liquid shampoo which entraps dirt without leaving any substantial residue, said shampoo comprising:
a hydrogel absorbent having as its active, dirt trapping ingredient carboxymethylcellulose cross-linked and stabilized by an organo-metallic cross-linking agent in an aqueous carrier which includes a cleansing agent having as its active ingredient a lauric acid derivative selected from lauric acid, sodium lauryl sulfate, ammonium lauryl sulfosuccinate, lauramide, lauramide diethanolamine, sodium lauryl betaine, or mixtures thereof; said aqueous carrier being at least 90% by weight water;
wherein said shampoo can be removed without need of addition rinse water.

2. The shampoo of claim 1 wherein said organo-metallic cross-linking agent comprises an aluminum complex.

3. The shampoo of claim 2 wherein said aluminum complex comprises aluminum acetate stabilized with boric acid.

## Patentansprüche

1. Ein flüssiges Shampoo, das Schmutz bindet, ohne irgendeinen wesentlichen Rückstand zu hinterlassen, wobei das Shampoo:
ein Hydrogel-Absorptionsmittel, das als seinen aktiven, schmutzbindenden Bestandteil Carboxymethylcellulose, vernetzt und stabilisiert durch ein organometallisches Vernetzungsmittel, in einem wäßrigen Träger enthält, der ein Reinigungsmittel enthält, das als seinen aktiven Bestandteil ein Laurinsäurederivat umfaßt, das aus Laurinsäure, Natriumlaurylsulfat, Ammoniumlaurylsulfosuccinat, Lauramid, Lauramiddiäthanolamin, Natriumlaurylbetain oder Mischungen derselben ausgewählt ist, umfaßt; wobei der wäßrige Träger wenigstens zu 90 Gewichts-% aus Wasser besteht;
wobei das Shampoo ohne die Notwendigkeit der Zugabe von Spülwasser entfernt werden kann.

2. Das Shampoo nach Anspruch 1, bei dem das organometallische Vernetzungsmittel einen Aluminiumkomplex umfaßt.

3. Das Shampoo nach Anspruch 2, bei dem der Aluminiumkomplex Aluminiumacetat, stabilisiert mit Borsäure, umfaßt.

## Revendications

1. Shampooing liquide piégeant les salissures sans laisser de résidu important, ce shampooing comprenant : un absorbant sous forme d'hydrogel comprenant, comme ingrédient actif de piégeage de salissure, de la carboxyméthylcellulose réticulée et stabilisée à l'aide d'un agent de réticulation organométallique dans un véhicule aqueux contenant un agent nettoyant comprenant, comme ingrédient actif, un dérivé d'acide laurique choisi parmi l'acide laurique, le laurylsulfate de sodium, le lauryl sulfosuccinate d'ammonium, un lauramide, la lauramide diéthanolamine, la lauryl bétaïne sodique ou des mélanges de ceux-ci, le véhicule aqueux comprenant au moins 90 % en poids d'eau ; ce shampooing pouvant être éliminé sans avoir besoin d'eau de rinçage supplémentaire.

2. Shampooing selon la revendication 1, l'agent de réticulation organo-métallique, comprenant un complexe d'aluminium.

3. Shampooing selon la revendication 2, le complexe d'aluminium comprenant de l'acétate d'aluminium stabilisé avec de l'acide borique.
